# EUROPÄISCHE PATENTSCHRIFT

(11) **EP 1 734 864 B1**
(45) Veröffentlichungstag und Bekanntmachung des Hinweises auf die Patenterteilung: **28.08.2013**
(21) Anmeldenummer: 05730820.7
(22) Anmeldetag: 01.04.2005
(51) Int. Cl.: A61B 5/15

(54) **VERFAHREN UND SYSTEM ZUM ENTNEHMEN VON KÖRPERFLÜSSIGKEIT**
METHOD AND SYSTEM FOR TAKING BODY FLUID
PROCEDE ET SYSTEME DE PRELEVEMENT DE LIQUIDES CORPORELS

(30) Priorität: 10.04.2004 EP 04008691
(43) Veröffentlichungstag der Anmeldung: 27.12.2006
(73) Patentinhaber: F.Hoffmann-La Roche AG, 4070 Basel (CH); Roche Diagnostics GmbH, 68305 Mannheim (DE)
(72) Erfinder: KORNER, Stephan, CH-6330 Cham (CH); JAEGGI, Rainer, CH-5610 Wohlen (CH); GRISS, Patrick, CH-8057 Zürich (CH); SAROFIM, Emad, CH-6300 Zug (CH); CALASSO, Irio Giuseppe, CH-6415 Arth (CH)
(74) Vertreter: Pfiz, Thomas
(86) Internationale Anmeldenummer: PCT/EP2005/003423
(87) Internationale Veröffentlichungsnummer: WO 2005/096941

(56) Entgegenhaltungen:
- EP-A- 1 304 075
- EP-A- 1 360 933
- WO-A-03/007819
- US-B1- 6 332 871

## Beschreibung

Die Erfindung betrifft Verfahren und ein System zum Entnehmen von Körperflüssigkeit, insbesondere Blut aus einem Körperteil gemäß dem Oberbegriff der unabhängigen Patentansprüche.

Solche Verfahren und Entnahmesysteme für kleine Körperflüssigkeitsmengen dienen vor allem Diabetikern zur täglich mehrfach durchgeführten Blutzucker-Selbstkontrolle im Rahmen einer normoglykämisch orientierten Insulinbehandlung. Damit auch von Laien die erforderlichen Schritte einfach und schnell vorgenommen werden können, ist es wünschenswert, so genannte integrierte Systeme zur Verfügung zu stellen, bei denen die Blutgewinnung und Analyse in einer Einheit kombiniert erfolgt. Für die Gewinnung von Kapillarblut ist es erforderlich, eine Hautöffnung durch einen Einstich zu erzeugen, wobei der Einstichschmerz und die Narbenbildung möglichst weitgehend reduziert werden sollen.

In der WO-A-01/89383 ist u. a. ein solches integriertes System beschrieben, bei dem eine elastomere Kompressionseinheit als Druckstück eingesetzt wird. Die Perforationsvorrichtung beinhaltet eine Kanüle, die in den komprimierten Hautbereich eingestochen wird und dort zum Sammeln von Körperflüssigkeit verbleibt. Neben dem Erfordernis eines ausreichenden Bluttransfers zu einer Analysezone kann es für den Benutzer auch in hygienischer und psychologischer Hinsicht problematisch sein, dass ein Teil des gewonnenen Blutes nach der Messung sichtbar auf der Hautoberfläche verbleibt. Ein ähnliches System ist auch in US 6332871 beschrieben

Ausgehend hiervon liegt der Erfindung die Aufgabe zugrunde, die im Stand der Technik aufgetretenen Nachteile zu vermeiden und bei einfacher Handhabung eine optimierte Gewinnung der Körperflüssigkeit zu ermöglichen.

Zur Lösung dieser Aufgabe wird die in den unabhängigen Patentansprüchen angegebene Merkmalskombination vorgeschlagen. Vorteilhafte Ausgestaltungen und Weiterbildungen der Erfindung ergeben sich aus den abhängigen Ansprüchen.

Die Erfindung geht von dem Gedanken aus, die Entnahme so durchzuführen, dass am Ende des Vorgangs möglichst keine Körperflüssigkeit, d.h. speziell Blut oder Gewebeflüssigkeit für den Anwender sichtbar ist. Dementsprechend wird in verfahrensmäßiger Hinsicht vorgeschlagen, dass das Druckstück zur Druckausübung relativ zu einer Abstützung für das Körperteil bewegt wird, und dass während (bevorzugt in der Endphase) der Flüssigkeitsentnahme die Druckkraft des Druckstücks auf das Körperteil erniedrigt wird, um das Austreten von Körperflüssigkeit zu vermeiden. Durch die Abstützung wird eine definierte Positionierung des daran anliegenden Körperteils für den Stechvorgang erreicht, während die Relativbewegung des Druckstücks eine zusätzliche Druckerhöhung und damit eine verbesserte Flüssigkeitsgewinnung ermöglicht. Ein zentraler Aspekt besteht darin, den durch Druckerhöhung unterstützten Flüssigkeitsfluss durch dauerhafte Druckreduzierung zu stoppen, so dass ein ungewollter bzw. überschüssiger Flüssigkeitsaustritt auf die Hautoberfläche vermieden wird. Dadurch ist nach der Entnahme auf der Hautoberfläche weitgehend keine Flüssigkeit bzw. Blut zu sehen. Dies ist in alltäglichen Umgebungen hygienischer und auch unter psychologischen Gesichtspunkten für den Anwender hilfreich. Vorteilhaft ist auch, dass die Stichwunde sich durch Erhöhen und Nachlassen der Druckkraft des Druckstücks öffnet und schließt. Damit wird auch das Schmerzempfinden reduziert, weil das Stechorgan an geeigneter Stelle in einer durch das Druckstück geöffneten Wunde verbleiben kann.

Vorteilhafterweise wird die Körperflüssigkeit über das Stechorgan als Teil der Sammeleinheit entnommen. Dabei sollte das Stechorgan während oder erst nach der Druckreduzierung aus dem Bereich des Körperteils weggezogen werden, so dass die Stichwunde nicht unter Flüssigkeitsdruck frei wird.

Eine besonders einfache Ausführung sieht vor, dass das Druckstück entgegen einer Widerstandskraft durch Andrücken des Körperteils auf die Abstützung zubewegt wird, wodurch das Körperteil zugleich komprimiert wird. Alternativ ist es auch möglich, dass das Druckstück durch einen Positionierantrieb an das gegen die Abstützung anliegende Körperteil herangefahren und/oder von diesem weggefahren wird.

In baulich einfacher Ausgestaltung lässt sich die Druckkraft des Druckstücks durch Entspannen eines gegen das Druckstück wirkenden Vorspannelements, insbesondere einer vorgespannten Feder erniedrigen. Denkbar ist es auch, ein beispielsweise als Tauchspule ausgebildetes Kraftelement zur Regulierung der Druckkraft einzusetzen.

Um auch dem Benutzer ein spürbares Signal für den Abschluss des Entnahmevorgangs zu vermitteln, ist es vorteilhaft, wenn das Druckstück am Ende der Flüssigkeitsentnahme von dem Körperteil wegbewegt wird. Eine weitere Verbesserung wird dadurch erreicht, dass das Druckstück zur Druckreduzierung durch eine Öffnung der Abstützung hindurch in eine gegen einen Eingriff des Körperteils abgeschirmte Stellung zurückbewegt wird.

Vorteilhafterweise wird durch das Druckstück ein freier Einstechbereich für das Stechorgan begrenzt.

Gemäß einer weiteren bevorzugten Variante bzw. einem Aspekt der Erfindung ist es für eine definierte Flüssigkeitsentnahme vorgesehen, dass die über das Druckstück ausgeübte Druckkraft gesteuert wird. Die Steuerung erfolgt dabei passiv durch eine gegebenenfalls benutzerdefinierte empirische Vorgabe der Entnahmedauer, oder aber aktiv durch eine sensorische Erfassung des Entnahmevolumens oder der momentanen Fließrate der Körperflüssigkeit. Letzteres ermöglicht eine rückgekoppelte Anpassung des Druckkraftverlaufs.

Eine definierte Ausgangskraft des Druckstücks lässt sich dadurch einstellen, dass bei Erreichen einer vorgegebenen Position des Druckstücks oder bei einer Berührung der Abstützung ein Freigabesignal zur Aktivierung des Stechvorgangs erzeugt wird. Möglich ist es auch, dass in einer Anschlagstellung des Druckstücks oder der Abstützung ein Freigabesignal zur Aktivierung des Stechvorgangs erzeugt wird. Die Aktivierung kann durch den Benutzer selbst erfolgen, oder aber automatisch durch einen Countdown nach dem Freigabesignal. Damit sollte in jedem Fall sichergestellt sein, dass der Stechvorgang nicht unerwartet für den Benutzer ausgelöst wird.

Um den Einstichschmerz während der Entnahme zu reduzieren, ist es vorteilhaft, wenn das Stechorgan vorzugsweise mit definierter Stechtiefe in das Körperteil eingestochen und dann in eine Entnahmeposition mit geringerer Stechtiefe zurückgezogen wird.

Eine besonders einfache Handhabung ergibt sich dadurch, dass zur Analyse der Körperflüssigkeit eine Detektionseinheit mit der Sammeleinheit gekoppelt wird. Hierbei ist es besonders günstig, wenn die Körperflüssigkeit über einen vorzugsweise kapillaren Transportkanal der Sammeleinheit aufgenommen und transportiert wird.

Zur anatomischen Anpassung an das jeweils beaufschlagte Körperteil können unterschiedliche Druckstücke wahlweise eingesetzt werden. Neben ringförmigen Druckstücken können auch andere, insbesondere linienförmige oder punktförmige Konturen gegebenenfalls in Kombination zum Einsatz kommen. Dadurch kann neben dem Druckaufbau auch eine genaue Positionierung des Körperteils unterstützt werden, und es kann die Blutgewinnung hinsichtlich der maßgeblichen Parameter zusätzlich kontrolliert werden. In dieser Hinsicht kann es auch von Vorteil sein, wenn das Druckstück vorzugsweise gummielastisch verformbar ausgebildet ist und insbesondere aus einem weichen oder elastomeren Material besteht. Auch dadurch wird eine verbesserte Anpassung des Druckstücks an das Körperteil ermöglicht.

Für die Flüssigkeitsgewinnung ist es vorteilhaft, wenn das Körperteil unter dem primären Druck des Druckstücks durch Querumlenkung in einem Einstechbereich ausgewölbt bzw. komprimiert oder aber gedehnt wird.

In vorrichtungsmäßiger Hinsicht wird die eingangs genannte Aufgabe dadurch gelöst, dass das Druckstück zur Druckausübung relativ zu einer Abstützung für das Körperteil beweglich ist, und dass während der Flüssigkeitsentnahme ein die Druckkraft reduzierender Aktuator mit dem Druckstück in Wirkverbindung steht.

Ein weiterer Erfindungsaspekt liegt darin, dass das Druckstück zur Druckausübung relativ zu einer Abstützung für das Körperteil beweglich ist, und dass eine Steuereinheit zur Druckkraftsteuerung des Druckstücks nach Maßgabe der Flüssigkeitsentnahme vorgesehen ist.

Weitere vorteilhafte Ausgestaltungen ergeben sich korrespondierend zu den vorstehenden Verfahrensmerkmalen und aus der nachfolgenden Erläuterung des in der Zeichnung in schematischer Weise dargestellten Ausführungsbeispiels. Es zeigen
- Fig. 1: eine Vorrichtung zum Entnehmen und Analysieren von Blut in einer blockschaltbildartigen Darstellung;
- Fig. 2: die prinzipielle Anordnung einer Stecheinheit, eines Druckrings und einer Positionierfläche der Vorrichtung nach Fig. 1 in perspektivischer Darstellung; und
- Fig. 3a bis h: verschiedene Stellungen der in Fig. 2 dargestellten Elemente bei der Blutentnahme in der Seitenansicht.

Die in der Zeichnung dargestellte Vorrichtung ist als Handgerät 10 zur kombinierten Blutentnahme und Analyse ausgebildet. Sie besteht im wesentlichen aus einem Gerätegehäuse 12 mit einer Abstützung 14 für ein Körperteil 16 eines Benutzers, einem mittels Aktuator 18 relativ zu der Abstützung 14 beweglichen Druckring 20, einer mit einem Stechorgan 22 versehenen Sammeleinheit 40 zur Blutentnahme und einer mit der Sammeleinheit 40 gekoppelten Detektionseinheit 24 zur Blutanalyse.

Die Stechbewegung der Sammeleinheit40 mit dem Stechorgan 22 erfolgt durch einen Stechantrieb 26 in einer Stechachse 28, welche durch eine Öffnung 32 der Abstützung 14 hindurch verläuft. Zur Ablaufsteuerung des Aktuators 18 und des Stechantriebs 26 sowie zur Auswertung der Messergebnisse ist eine Prozessoreinheit 30 vorgesehen.

Der Druckring 20 ist durch eine Linearführung 24 koaxial zu dem Stechorgan 22 in der Stechachse 28 beweglich. Dabei kann der Druckring 20 durch Andrücken des Körperteils 16 entgegen der Kraft einer Rückstellfeder 36 des Aktuators 18 zurückgedrückt werden, so dass als Reaktion der Druck auf das Körperteil während der Blutentnahme erhöht wird. Um anschließend in der Endphase der Blutentnahme eine Druckreduzierung zu ermöglichen, weist der Aktuator eine bewegliche Federstütze 38 zum Entspannen des Federelements 36 auf. Alternativ ist es auch möglich, dass der Aktuator 18 einen beispielsweise kurvengesteuerten Positionierantrieb zur Steuerung des Bewegungsablaufs des Druckrings 20 aufweist (nicht gezeigt).

Zum selbsttätigen Transport der an der Einstichstelle entnommenen Blutflüssigkeit zu der Detektionseinheit 24 weist die Sammeleinheit 40 einen kapillaren Transportkanal auf. Dieser kann durch eine randoffene Kapillarrille gebildet sein, um die Herstellung zu vereinfachen. Zur Erfassung des Glucosegehalts im Blut ist die Detektionseinheit 24 beispielsweise als reflexionsphotometrische Einheit auf ein geeignetes Testfeld ausgerichtet. Solche Meßsysteme mit disposiblen Testelementen zur Blutzuckerselbstkontrolle für Diabetiker sind an sich bekannt.

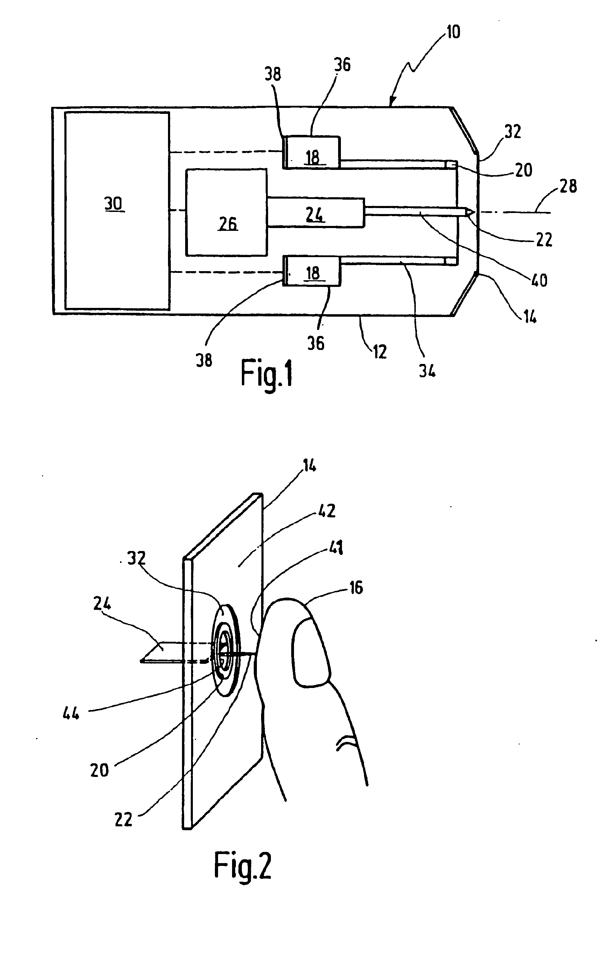

In Fig. 2 ist die prinzipielle Anordnung für die direkte Blutgewinnung aus dem Körperteil 16 veranschaulicht. Bevorzugt erfolgt die Blutentnahme aus der Fingerbeere 41, die gegen die zugewandte Positionierfläche 42 der Abstützung 14 im Bereich der Öffnung 32 angelegt wird. Der Druckring 20 ist dabei durch die Öffnung 32 hindurch verschiebbar, während das Stechorgan 22 als kapillaraktive Nadelspitze durch die Ringöffnung 44 in die Fingerbeere 41 einstechbar ist.

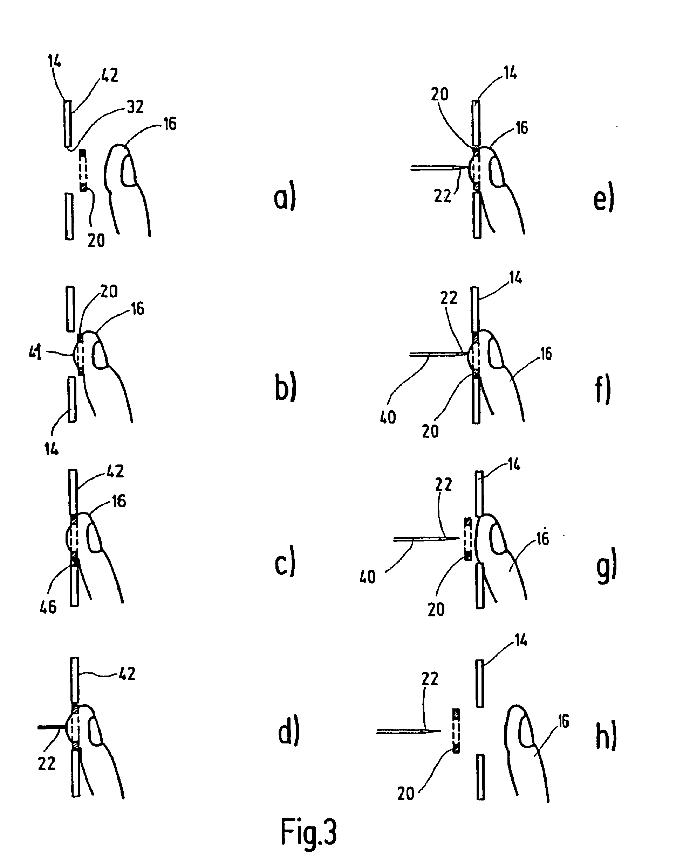

Fig. 3 zeigt die besondere Abfolge der einzelnen Verfahrensschritte bei der Blutgewinnung. Entsprechend Fig. 3a und b wird das aus dem Gerätegehäuse ausgefahrene Druckstück 20 entgegen einer Widerstandskraft durch Andrücken des Körperteils 16 in Richtung der Abstützung 14 bewegt. Unter dem erhöhten Druck wird das Körperteil 16 im Bereich der Fingerbeere bzw. Fingerkuppe 41 ausgewölbt, so dass sich zusätzlich Blut ansammelt.

Bei Erreichen der Positionierfläche 42 wird durch einen Berührungssensor 46 ein Freigabesignal erzeugt (Fig. 3c), woraufhin der Benutzer den Stechvorgang auslöst. Es erfolgt ein schnelles Hineinstechen des Stechorgans 22 mit definierter Stechtiefe (Fig. 3d) und eine Rückbewegung bzw. Herausziehen in eine Entnahmeposition mit geringerer Stechtiefe (Fig. 3e). In dieser Position befindet sich das Stechorgan im Bereich der Haut (gegebenenfalls auch knapp darüber), während der offene Stichkanal sich mit Blut füllt. Dabei strömt das Blut aufgrund von Kapillarkräften selbsttätig in den Transportkanal (Fig. 3f). Eventuell wird in dieser Phase die Gegenkraft durch den Ring 20 moduliert bzw. variiert, um den Blutfluss zu steuern.

Nach Entnahme einer definierten Blutmenge, die aktiv erfasst oder empirisch anhand einer zugeordneten Entnahmedauer ermittelt werden kann, wird die Druckkraft des Druckrings 20 stetig reduziert, um einen Blutaustritt aus der Stichwunde zu verhindern ("no blood seen"). Zu diesem Zweck wird der Druckring 20 durch die Öffnung 32 der Abstützung 14 hindurch in eine gegen den Eingriff des Körperteils 16 abgeschirmte Gehäusestellung zurückgefahren. Dabei wird auch die Sammeleinheit 40 vollständig aus dem Bereich des Körperteils 16 weggezogen (Fig. 3g), wodurch die Blutaufnahme beendet wird. In der in Fig. 3h gezeigten Endposition steht der Druckring 20 gehäuseintern hinter der Abstützung 14. Die Sammeleinheit 40 ist ebenfalls in das Gehäuse zurückgezogen, so dass die Analyse durchgeführt werden kann. Das Wegfahren des Druckrings 20 von dem Körperteil 16 ist auch für den Benutzer ein fühlbares Signal für das Ende des Entnahmevorgangs.

## Patentansprüche

1. Verfahren zum Entnehmen von Körperflüssigkeit, insbesondere Blut aus einem Körperteil (16), bei dem Druck auf das Körperteil (16) durch ein Druckstück (20) ausgeübt wird, ein Stechorgan (22) in das Körperteil (16) eingestochen wird und Körperflüssigkeit durch eine Sammeleinheit (40) entnommen wird, **dadurch gekennzeichnet, dass** das Druckstück (20) zur Druckausübung relativ zu einer Abstützung (14) für das Körperteil (16) bewegt wird, und dass während der Flüssigkeitsentnahme die Druckkraft des Druckstücks (20) auf das Körperteil (16) erniedrigt wird, um das Austreten von Körperflüssigkeit zu vermeiden.

2. Verfahren nach Anspruch 1, **dadurch gekennzeichnet, dass** die Druckkraft des Druckstücks (20) in der Endphase der Flüssigkeitsentnahme erniedrigt wird.

3. Verfahren nach Anspruch 1 oder 2, **dadurch gekennzeichnet, dass** die Körperflüssigkeit über das Stechorgan (22) als Teil der Sammeleinheit (40) entnommen wird.

4. Verfahren nach einem der Ansprüche 1 bis 3, **dadurch gekennzeichnet, dass** das Stechorgan (22) während oder nach der Druckreduzierung von dem Körperteil (16) entfernt wird.

5. Verfahren nach einem der Ansprüche 1 bis 4, **dadurch gekennzeichnet, dass** das Druckstück (20) entgegen einer Widerstandskraft durch Andrücken des Körperteils (16) in Richtung der Abstützung (14) bewegt wird.

6. Verfahren nach einem der Ansprüche 1 bis 5, **dadurch gekennzeichnet, dass** das Druckstück (20) durch einen Positionierantrieb an das gegen die Abstützung (14) anliegende Körperteil (16) herangefahren und/oder von diesem weggefahren wird.

7. Verfahren nach einem der Ansprüche 1 bis 6, **dadurch gekennzeichnet, dass** die Druckkraft des Druckstücks (20) durch Entspannen eines gegen das Druckstück (20) wirkenden Vorspannelements (36), insbesondere einer vorgespannten Feder erniedrigt wird.

8. Verfahren nach einem der Ansprüche 1 bis 6, **gekennzeichnet durch** ein beispielsweise als Tauchspule ausgebildetes Kraftelement zur Regulierung der Druckkraft des Druckstücks (20).

9. Verfahren nach einem der Ansprüche 1 bis 8, **dadurch gekennzeichnet, dass** das Druckstück (20) am Ende der Flüssigkeitsentnahme von dem Körperteil (16) wegbewegt wird.

10. Verfahren nach einem der Ansprüche 1 bis 9, **dadurch gekennzeichnet, dass** das Druckstück (20) zur Druckreduzierung durch eine Öffnung (32) der Abstützung (14) hindurch in eine gegen einen Eingriff des Körperteils (16) abgeschirmte Stellung zurückbewegt wird.

11. Verfahren nach einem der Ansprüche 1 bis 10, **dadurch gekennzeichnet, dass** durch das Druckstück (20) ein freier Einstechbereich für das Stechorgan (22) begrenzt wird.

12. Verfahren nach einem der Ansprüche 1 bis 11, **dadurch gekennzeichnet, dass** die über das Druckstück (20) ausgeübte Druckkraft für eine definierte Flüssigkeitsentnahme gesteuert wird.

13. Verfahren nach Anspruch 12, **dadurch gekennzeichnet, dass** die Steuerung durch eine gegebenenfalls benutzerdefinierte Vorgabe der Entnahmedauer oder durch eine Erfassung des Entnahmevolumens oder der momentanen Fließrate der Körperflüssigkeit erfolgt.

14. Verfahren nach einem der Ansprüche 1 bis 13, **dadurch gekennzeichnet, dass** bei Erreichen einer vorgegebenen Position des Druckstücks (20) oder bei einer Berührung der Abstützung (14) ein Freigabesignal zur Aktivierung des Stechvorgangs erzeugt wird.

15. Verfahren nach einem der Ansprüche 1 bis 14, **dadurch gekennzeichnet, dass** in einer Anschlagstellung des Druckstücks (20) oder der Abstützung (14) ein Freigabesignal zur Aktivierung des Stechvorgangs erzeugt wird.

16. Verfahren nach einem der Ansprüche 1 bis 15, **dadurch gekennzeichnet, dass** das Stechorgan (22) vorzugsweise mit definierter Stechtiefe in das Körperteil (16) eingestochen und dann in eine Entnahmeposition in geringerer Stechtiefe zurückgezogen wird.

17. Verfahren nach einem der Ansprüche 1 bis 16, **dadurch gekennzeichnet, dass** zur Analyse der Körperflüssigkeit eine Detektionseinheit (24) mit der Sammeleinheit (40) gekoppelt wird.

18. Verfahren nach einem der Ansprüche 1 bis 17, **dadurch gekennzeichnet, dass** die Körperflüssigkeit über einen vorzugsweise kapillaren Transportkanal der Sammeleinheit (40) aufgenommen wird.

19. Verfahren nach einem der Ansprüche 1 bis 18, **dadurch gekennzeichnet, dass** unterschiedliche Druckstücke (20) zur anatomischen Anpassung an das Körperteil (16) wahlweise eingesetzt werden.

20. Verfahren nach einem der Ansprüche 1 bis 19, **dadurch gekennzeichnet, dass** das Körperteil (16) unter dem Druck des Druckstücks (20) in einem Einstechbereich (41) ausgewölbt oder gedehnt wird.

21. System zum Entnehmen von Körperflüssigkeit, insbesondere Blut aus einem Körperteil (16) mit einem Druckstück (20) zur Druckbeaufschlagung des Körperteils (16), einem in das Körperteil (16) einstechbaren Stechorgan (22) und einer Sammeleinheit (40) zur Flüssigkeitsentnahme, wobei das Druckstück (20) zur Druckausübung relativ zu einer Abstützung (14) für das Körperteil (16) beweglich ist, **dadurch gekennzeichnet, dass** während der Flüssigkeitsentnahme ein die Druckkraft reduzierender Aktuator (18) mit dem Druckstück (20) in Wirkverbindung steht, um durch die Druckkraftreduzierung ein Austreten von überschüssiger Körperflüssigkeit zu vermeiden.

22. System nach Anspruch 21, **dadurch gekennzeichnet, dass** das Stechorgan (22) an der Sammeleinheit (40) ausgebildet ist.

23. System nach Anspruch 21 oder 22, **dadurch gekennzeichnet, dass** das Druckstück (20) durch Andrücken des Körperteils (16) entgegen einer Rückstellkraft des Aktuators (18) in Richtung der Abstützung (14) beweglich ist.

24. System nach einem der Ansprüche 21 bis 23, **dadurch gekennzeichnet, dass** der Aktuator (18) ein unter Vorspannung gegen das Druckstück (20) anliegendes Vorspannelement (36), insbesondere eine Feder aufweist.

25. System nach einem der Ansprüche 21 bis 23, **dadurch gekennzeichnet, dass** der Aktuator (18) eine bewegliche Stütze (38) zum Entspannen des Vorspannelements (36) aufweist.

26. System nach Anspruch 21, **dadurch gekennzeichnet, dass** der Aktuator (18) einen insbesondere kurvengesteuerten Positionierantrieb zur Bewegung des Druckstücks (20) aufweist.

27. System nach einem der Ansprüche 21 bis 26, **dadurch gekennzeichnet, dass** die Abstützung (14) eine mit einer Öffnung (32) für den Durchtritt des Druckstücks (20) versehene Positionierfläche (42) zum Anlegen des Körperteils (16) aufweist.

28. System nach einem der Ansprüche 21 bis 27, **dadurch gekennzeichnet, dass** das Druckstück (20) durch einen Ring gebildet ist, und dass die Stecheinheit (22) durch die Ringöffnung (44) hindurch in das Körperteil (16) einstechbar ist.

29. System nach einem der Ansprüche 21 bis 28, **dadurch gekennzeichnet, dass** das Druckstück (20) durch mindestens einen linienförmig andrückenden Steg oder punktförmig andrückenden Stiel gebildet ist.

30. System nach einem der Ansprüche 21 bis 29, **dadurch gekennzeichnet, dass** das Druckstück (20) vorzugsweise gummielastisch verformbar ausgebildet ist und insbesondere aus einem weichen oder elastomeren Material besteht.

31. System nach einem der Ansprüche 21 bis 30, **gekennzeichnet durch** einen bei Erreichen einer vorgegebenen Position oder bei einer Berührung oder Anschlagstellung der Abstützung (14) oder des Druckstücks (20) ansprechenden Signalgeber (46) zur Erzeugung eines Freigabesignals für die Aktivierung der Stecheinheit (22).

32. System nach einem der Ansprüche 21 bis 31, **dadurch gekennzeichnet, dass** die Sammeleinheit (40) mit einer Detektionseinheit (24) zur Analyse der Körperflüssigkeit in Wirkverbindung steht.

33. System nach einem der Ansprüche 21 bis 32, **dadurch gekennzeichnet, dass** die Sammeleinheit (40) einen vorzugsweise kapillaren Transportkanal zum Aufnehmen der Körperflüssigkeit aufweist.

## Claims

1. Method for withdrawing body fluid, in particular blood, from a body part (16) in which pressure is exerted on the body part (16) by a pressure piece (20), a lancing member (22) is inserted into the body part (16) and body fluid is withdrawn by a collecting unit (40), **characterized in that** the pressure piece (20) is moved relative to a support (14) for the body part (16) in order to exert pressure, and that during the fluid withdrawal the compressive force of the pressure piece (20) on the body part (16) is reduced in order to avoid the escape of body fluid.

2. Method according to claim 1, **characterized in that** the compressive force of the pressure piece (20) is lowered in the final phase of fluid withdrawal.

3. Method according to claim 1 or 2, **characterized in that** the body fluid is withdrawn by the lancing member (22) as part of the collecting unit (40).

4. Method according to one of the claims 1 to 3, **characterized in that** the lancing member (22) is removed from the body part (16) during or after the pressure reduction.

5. Method according to one of the claims 1 to 4, **characterized in that** the pressure piece (20) is moved towards the support (14) against a resisting force by pressing on the body part (16).

6. Method according to one of the claims 1 to 5, **characterized in that** the pressure piece (20) is moved towards and/or moved away from the body part (16) resting against the support (14) by means of a positioning drive.

7. Method according to one of the claims 1 to 6, **characterized in that** the compressive force of the pressure piece (20) can be lowered by relaxing a pretensioning element (36), in particular a pretensioned spring acting against the pressure piece (20).

8. Method according to one of the claims 1 to 6, **characterized by** a force element designed for example as a plunger coil to regulate the compressive force of the pressure piece (20).

9. Method according to one of the claims 1 to 8, **characterized in that** the pressure piece (20) is moved away from the body part (16) at the end of fluid withdrawal.

10. Method according to one of the claims 1 to 9, **characterized in that** the pressure piece (20) is moved back through an opening (32) of the support (14) into a position that is screened from contact by the body part (16) in order to reduce the pressure.

11. Method according to one of the claims 1 to 10, **characterized in that** the pressure piece (20) delimits a free puncture area for the lancing member (22).

12. Method according to one of the claims 1 to 11, **characterized in that** the compressive force exerted by the pressure piece (20) is controlled for a defined fluid withdrawal.

13. Method according to claim 12, **characterized in that** the control occurs by an optionally user-defined presetting for the withdrawal duration or by a detection of the collected volume or of the actual flow rate of the body fluid.

14. Method according to one of the claims 1 to 13, **characterized in that** a release signal to activate the lancing process is generated when the pressure piece (20) reaches a preset position or when the support (14) is touched.

15. Method according to one of the claims 1 to 14, **characterized in that** in a stop position of the pressure piece (20) or of the support (14) a release signal is generated to activate the lancing process.

16. Method according to one of the claims 1 to 15, **characterized in that** the lancing member (22) is inserted preferably to a defined lancing depth into the body part (16) and then is retracted into a collecting position at a smaller lancing depth.

17. Method according to one of the claims 1 to 16, **characterized in that** a detection unit (24) is coupled with the collecting unit (40) to analyse the body fluid.

18. Method according to one of the claims 1 to 17, **characterized in that** the body fluid is taken up by a preferably capillary transport channel of the collecting unit (40).

19. Method according to one of the claims 1 to 18, **characterized in that** different pressure pieces (20) are optionally used for an anatomical adaptation to the body part (16).

20. Method according to one of the claims 1 to 19, **characterized in that** the body part (16) is bulged or stretched in a puncture area (41) under the pressure of the pressure piece (20).

21. System for withdrawing body fluid, in particular blood, from a body part (16) comprising a pressure piece (20) to apply pressure to the body part (16), a lancing member (22) that can be inserted into the body part (16) and a collecting unit (40) to withdraw fluid, wherein the pressure piece (20) can be moved relative to a support (14) for the body part (16) in order to exert pressure, **characterized in that** during the fluid withdrawal an actuator (18) reducing the compressive force is in operative connection with the pressure piece (20) in order to avoid escape of excessive body fluid onto the skin surface by the reduction of compressive force.

22. System according to claim 21, **characterized in that** the lancing member (22) is formed on the collecting unit (40).

23. System according to claim 21 or 22, **characterized in that** the pressure piece (20) can be moved towards the support (14) against a restoring force of the actuator (18) by pressing on the body part (16).

24. System according to one of the claims 21 to 23, **characterized in that** the actuator (18) has a pretensioning element (36) and in particular a spring resting under pretension against the pressure piece (20).

25. System according to one of the claims 21 to 23, **characterized in that** the actuator (18) has a movable support (38) to relax the pretensioning element (36).

26. System according to claim 21, **characterized in that** the actuator (18) has an in particular curve-controlled positioning drive for the movement of the pressure piece (20).

27. System according to one of the claims 21 to 26, **characterized in that** the support (14) has a positioning surface (42) to apply the body part (16) said positioning surface being provided with an opening (32) for the passage of the pressure piece (20).

28. System according to one of the claims 21 to 27, **characterized in that** the pressure piece (20) is formed by a ring and that the lancing unit (22) can be lanced through the ring opening (44) into the body part (16).

29. System according to one of the claims 21 to 28, **characterized in that** the pressure piece (20) is formed by at least one bar that presses down linearly or by a strut that presses down in a punctiform manner.

30. System according to one of the claims 21 to 29, **characterized in that** when the pressure piece (20) is preferably deformable in a rubber elastic manner and in particular consists of a soft or elastomeric material.

31. System according to one of the claims 21 to 30, **characterized by** a signal generator (46) that is activated when a preset position is reached or when the support (14) or the pressure piece (20) is touched or reaches a stop position in order to generate a release signal for the activation of the lancing unit (22).

32. System according to one of the claims 21 to 31, **characterized in that** the collecting unit (40) is in operative connection with a detection unit (24) to analyse the body fluid.

33. System according to one of the claims 21 to 32, **characterized in that** the collecting unit (40) has a preferably capillary transport channel to take up the body fluid.

## Revendications

1. Procédé de prélèvement de liquide corporel, notamment du sang provenant d'une partie du corps (16), dans lequel une pression est exercée sur la partie du corps (16) par une pièce de pression (20), un organe de piqûre (22) est enfoncé dans la partie du corps (16) et du liquide corporel est prélevé par le biais d'une unité de collecte (40), **caractérisé en ce que**, pour exercer une pression, la pièce de pression (20) est déplacée par rapport à un support (14) pour la partie du corps (16) et **en ce que** pendant le prélèvement de liquide la force de pression de la pièce de pression (20) sur la partie du corps (16) est réduite afin d'éviter que du liquide corporel ne s'échappe.

2. Procédé selon la revendication 1, **caractérisé en ce que** la force de pression de la pièce de pression (20) est réduite lors de la phase terminale du prélèvement de liquide.

3. Procédé selon la revendication 1 ou 2, **caractérisé en ce que** le liquide corporel est prélevé par le biais de l'organe de piqûre (22) en tant que partie de l'unité de collecte (40).

4. Procédé selon l'une des revendications 1 à 3, **caractérisé en ce que** l'organe de piqûre (22) est éloigné de la partie du corps (16) pendant ou après la réduction de pression.

5. Procédé selon l'une des revendications 1 à 4, **caractérisé en ce que** la pièce de pression (20) est déplacée à l'encontre d'une force de résistance par le biais d'une pression de la partie de corps (16) dans la direction du support (14).

6. Procédé selon l'une des revendications 1 à 5, **caractérisé en ce que** la pièce de pression (20) est approchée et/ou éloignée de la partie du corps (16) se trouvant contre le support (14) par le biais d'un mécanisme d'entraînement en position.

7. Procédé selon l'une des revendications 1 à 6, **caractérisé en ce que** la force de pression de la pièce de pression (20) est réduite par le biais d'une détente d'un élément de précontrainte (36), notamment un ressort précontraint, agissant contre la pièce de pression (20).

8. Procédé selon l'une des revendications 1 à 6, **caractérisé par** un élément de force configuré par exemple en tant que bobine mobile destiné à réguler la force de pression de la pièce de pression (20).

9. Procédé selon l'une des revendications 1 à 8, **caractérisé en ce que** la pièce de pression (20) est éloignée de la partie du corps (16) à la fin du prélèvement de liquide.

10. Procédé selon l'une des revendications 1 à 9, **caractérisé en ce que** pour la réduction de la pression, la pièce de pression (20) est ramenée à travers une ouverture (32) du support (14) dans une position protégée contre une atteinte de la partie du corps (16).

11. Procédé selon l'une des revendications 1 à 10, **caractérisé en ce que** par le biais de la pièce de pression (20) une zone de piqûre libre est délimitée pour l'organe de piqûre (22).

12. Procédé selon l'une des revendications 1 à 11, **caractérisé en ce que** la force de pression exercée par la pièce de pression (20) est commandée pour un prélèvement de liquide défini.

13. Procédé selon la revendication 12, **caractérisé en ce que** la commande s'effectue par le biais d'une instruction de la durée de prélèvement le cas échéant définie par l'utilisateur ou par le biais d'une détection du volume de prélèvement ou du débit momentané du liquide corporel.

14. Procédé selon l'une des revendications 1 à 13, **caractérisé en ce que** lorsqu'une position prédéfinie de la pièce de pression (20) est atteinte ou lors d'un contact du support (14) un signal de libération destiné à l'activation du processus de piqûre est généré.

15. Procédé selon l'une des revendications 1 à 14, **caractérisé en ce que** dans une position de butée de la pièce de pression (20) ou du support (14) un signal de libération destiné à l'activation du processus de piqûre est généré.

16. Procédé selon l'une des revendications 1 à 15, **caractérisé en ce que** l'organe de piqûre (22) est de préférence enfoncé dans la partie du corps (16) selon une profondeur de piqûre définie et est ensuite ramené dans une position de prélèvement à une profondeur de piqûre moindre.

17. Procédé selon l'une des revendications 1 à 16, **caractérisé en ce que** pour l'analyse du liquide corporel une unité de détection (24) est couplée à l'unité de collecte (40).

18. Procédé selon l'une des revendications 1 à 17, **caractérisé en ce que** le liquide corporel est reçu par un canal de transport de préférence capillaire de l'unité de collecte (40).

19. Procédé selon l'une des revendications 1 à 18, **caractérisé en ce que** différentes pièces de pression (20) sont utilisées au choix pour l'adaptation anatomique à la partie du corps (16).

20. Procédé selon l'une des revendications 1 à 19, **caractérisé en ce que** la partie du corps (16) est bombée ou allongée sous la pression de pièce de pression (20) dans une zone de piqûre (41).

21. Système de prélèvement de liquide corporel, notamment du sang provenant d'une partie du corps (16), avec une pièce de pression (20) destinée à l'application d'une pression sur la partie du corps (16), un organe de piqûre (16) qui peut être enfoncé dans la partie du corps (16) et une unité de collecte (40) destinée au prélèvement de liquide, la pièce de pression (20) étant mobile pour exercer une pression par rapport à un support (14) pour la partie du corps (16), **caractérisé en ce que** pendant le prélèvement de liquide un actionneur (18) réduisant la force de pression est en liaison active avec la pièce de pression (20), afin d'éviter par le biais d'une réduction de la force de pression l'apparition d'un excédent de liquide corporel.

22. Système selon la revendication 21, **caractérisé en ce que** l'organe de piqûre (22) est configuré au niveau de l'unité de collecte (40).

23. Système selon la revendication 21 ou 22, **caractérisé en ce que** la pièce de pression (20) est mobile par le biais d'une poussée de la partie du corps (16) à l'encontre d'une force de rappel de l'actionneur (18) dans la direction du support (14).

24. Système selon l'une des revendications 21 à 23, **caractérisé en ce que** l'actionneur (18) présente un élément de précontrainte (36), notamment un ressort, qui se trouve contre la pièce de pression (20) sous l'effet d'une précontrainte.

25. Système selon l'une des revendications 21 à 23, **caractérisé en ce que** l'actionneur (18) présente un appui mobile (38) destiné à la détente de l'élément de précontrainte (36).

26. Système selon la revendication 21, **caractérisé en ce que** l'actionneur (18) présente un mécanisme d'entraînement en position notamment à commande par cames destiné au déplacement de la pièce de pression (20).

27. Système selon l'une des revendications 21 à 26, **caractérisé en ce que** le support (14) présente une surface de positionnement (42) destinée à disposer la partie du corps (116) et pourvue d'une ouverture (32) pour la traversée de la pièce de pression (20).

28. Système selon l'une des revendications 21 à 27, **caractérisé en ce que** la pièce de pression (20) est formée par une bague, et **en ce que** l'unité de piqûre (22) peut être enfoncée à travers l'ouverture de bague (44) dans la partie du corps (16).

29. Système selon l'une des revendications 21 à 28, **caractérisé en ce que** la pièce de pression (20) est formée par au moins une arête exerçant une pression de manière linéaire ou une tige exerçant une pression à la manière d'un point.

30. Système selon l'une des revendications 21 à 29, **caractérisé en ce que** la pièce de pression (20) est configurée de préférence de manière a être déformable élastiquement comme du caoutchouc et se compose notamment d'un matériau souple ou élastomérique.

31. Système selon l'une des revendications 21 à 30, **caractérisé par** un dispositif émetteur de signaux (46) réagissant lorsqu'une position prédéfinie est atteinte ou lors d'un contact ou d'une position de butée du support (14) ou de la pièce de pression (20) destiné à la génération d'un signal de libération pour l'activation de l'unité de piqûre (22).

32. Système selon l'une des revendications 21 à 31, **caractérisé en ce que** l'unité de collecte (40) est en liaison active avec une unité de détection (24) pour l'analyse du liquide corporel.

33. Système selon l'une des revendications 21 à 32, **caractérisé en ce que** l'unité de collecte (40) présente un canal de transport de préférence capillaire destiné à recevoir le liquide corporel.
